(19) Europäisches Patentamt / European Patent Office / Office européen des brevets

(11) **EP 2 301 521 A2**

(12) **EUROPÄISCHE PATENTANMELDUNG**

(43) Veröffentlichungstag:
**30.03.2011 Patentblatt 2011/13**

(51) Int Cl.:
*A61K 8/44* (2006.01)    *A61Q 5/10* (2006.01)

(21) Anmeldenummer: **10171740.3**

(22) Anmeldetag: **03.08.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **23.09.2009   DE 102009044920**

(71) Anmelder: **Henkel AG & Co. KGaA**
**40589 Düsseldorf (DE)**

(72) Erfinder:
• **Reichert, Anja**
  **40629, Düsseldorf (DE)**
• **Rohland, Christa**
  **40468, Düsseldorf (DE)**
• **Kleen, Astrid**
  **20457, Hamburg (DE)**

(54) **Verwendung bestimmter Aminosäuren in Färbemitteln keratinischer Fasern zur Farbintensivierung**

(57)    Die vorliegende Anmeldung betrifft die kosmetische Verwendung eines Mittels zur Intensivierung der Farbveränderung keratinischer Fasern, wobei das Mittel in einem kosmetischen Träger mindestens eine farbverändernde Komponente und mindestens eine Aminosäure enthält. Weiterhin wird eine Verpackungseinheit, umfassend solche Färbezubereitungen, offenbart. Dadurch ist eine deutlich verbesserte Farbintensivierung gegenüber üblichen Färbeverfahren erzielbar.

EP 2 301 521 A2

**Beschreibung**

Die Erfindung betrifft die kosmetische Verwendung von Färbemittel für keratinhaltige Fasern, insbesondere menschliche Haare, wobei die Mittel neben einer farbverändernden Komponente mindestens eine Aminosäure enthalten, zur Intensivierung der Farbveränderung. Weiterhin betrifft die vorliegende Erfindung eine Verpackungseinheit, welche eine Färbezubereitung, enthaltend Oxidationsfarbstoffvorprodukte, mindestens ein amphoteres oder kationisches Polymer und mindestens eine hydroxylgruppen-haltige Aminosäure und/oder deren Salze, und eine Oxidationszubereitung umfasst.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und die Faserstruktur schonen. Um die Schädigung weiterhin zu reduzieren, ist es notwendig, dass die Mittel durch Zusätze in ihrer Färbeleistung möglichst verbessert werden, so dass intensivere und lang anhaltende Farben erzielt werden, ohne die Mengen an Färbemittel zu erhöhen.

Zur Bereitstellung farbverändernder kosmetischer Mittel, insbesondere für die Haut oder keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen.

Eine weitere Möglichkeit zur Farbveränderung bietet die Verwendung von Färbemitteln, die sogenannte Oxofarbstoffvorprodukte, die sich in zwei Klassen unterteilen, enthalten. Eine erste Klasse der Oxofarbstoffvorprodukte sind Verbindungen mit mindestens einer reaktiven Carbonylgruppe. Eine zweite Klasse der Oxofarbstoffvorprodukte bilden C,H-acide Verbindungen und Verbindungen mit primärer oder sekundärer Aminogruppe oder Hydroxygruppe, vorzugsweise aromatischen Verbindungen. Die vorgenannten Komponenten sind im Allgemeinen selbst keine Farbstoffe, und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind, ohne jedoch auf Oxidationsmittel zur Farbstoffausbildung angewiesen zu sein.

Bei einem weiteren Färbeverfahren werden Vorstufen des natürlichen Haarfarbstoffes Melanin auf das Haar aufgebracht, die dann im Rahmen oxidativer Prozesse im Haar naturanaloge Farbstoffe aus bilden. Die Ausfärbung kann dabei mit Luftsauerstoff als einzigem Oxidationsmittel erfolgen, so dass auf keine weiteren Oxidationsmittel zurückgegriffen werden muss.

Insbesondere oxidative Haarfärbemittel sind trotz ihrer vorteilhaften Färbeeigenschaften für den Anwender mit Nachteilen behaftet. Insbesondere der Einsatz der Oxidationsmittel zur Ausfärbung respektive Entwicklung der eigentlichen Färbung führt zu Schädigungen in der Haarstruktur und auf der Haaroberfläche. Eine Verbesserung der Färbeleistung eines Mittels hinsichtlich Farbintensität würde eine Reduktion von Anwendungsmenge oder Anwendungsdauer erlauben.

Aufgabe der vorliegenden Erfindung ist es daher, die Intensität und Leuchtkraft der Färbungen von Haarfärbemitteln zu verbessern.

Es wurde nun in nicht vorhersehbarer Weise gefunden, dass durch die Verwendung von farbverändernden Mitteln, welche zusätzlich eine Aminosäure enthalten, eine signifikante Verbesserung der Farbintensität erzielt werden kann.

Ein erster Gegenstand der vorliegenden Erfindung ist daher die kosmetische Verwendung eines Mittels zur Intensivierung der Farbveränderung keratinischer Fasern, welche dadurch gekennzeichnet ist, dass das Mittel in einem kosmetischen Träger mindestens eine farbverändernde Komponente und mindestens eine Aminosäure enthält.

Unter der Verbesserung der Farbintensität ist im Sinne der Erfindung insbesondere die Verbesserung des sogenannten ΔE-Wertes zu verstehen. Der ΔE-Wert kann mittels üblicher Lab-Farbraumbestimmungen bestimmt werden, die dem Fachmann wohl bekannt sind.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Als ersten wesentlichen Inhaltsstoff enthält die Färbezubereitung zur erfindungsgemäßen Verwendung mindestens eine farbverändernde Komponente.

Die farbverändernde Komponente wird dabei ausgewählt aus mindestens einem

(a) Oxidationsfarbstoffvorprodukt und/oder
(b) direktziehenden Farbstoff und/oder
(c) naturanalogen Farbstoff und/oder
(d) Oxofarbstoffvorprodukt.

In einer bevorzugten Ausführungsform der erfindungsgemäßen Verwendung enthält die Färbezubereitung als farbverändernde Komponente mindestens ein Oxidationsfarbstoffvorprodukt.

Als Oxidationsfarbstoffvorprodukte enthalten die Färbezubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Di-aminobenzo-1,4-dioxan sowie ihren physiologisch verträglichen Salzen. Erfindungsgemäß bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salzen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendi-amin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-amino-benzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diamino-phenoxy)-propan-2-ol, N,N'-Bis-(4-aminophenyl)-1,4-diazacycloheptan, 1,10-Bis-(2,5-diamino-phenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Di-amino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1 -methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]pyrimidine bevorzugt, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Amino-pyrazolo[1,5-a]pyrimidin-7-ol, 3-Aminopyrazolo[1,5-a]pyrimidin-5-ol, 2-(3-Aminopyrazolo[1,5-a]-pyrimidin-7-ylamino)ethanol, 2-(7-Aminopyrazolo[1,5-a]pyrimidin-3-ylamino)ethanol, 2-[(3-Amino-pyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Di-methylpyrazolo[1,5-a]pyrimidin-3,7-diamin, 3-Amino-7-dimethylamino-2,5-dimethylpyrazolo[1,5-a]-pyrimidin sowie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydro-chinoxalin, und/oder Gemischen aus zwei oder mehreren Verbindungen aus einer oder mehreren dieser Klassen.

Die erfindungsgemäß verwendbaren m-Aminophenole bzw. deren Derivate werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Di-hydroxy-5-(methylamino)benzol, 3-Ethylamino-4-methylphenol, 2,4-Dichlor-3-aminophenol und deren physiologisch verträglichen Salzen.

Die erfindungsgemäß verwendbaren 3-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxy-pyridin, 2-Amino-5-chlor-3-hydroxypyridin, 3-Amino-2-methylamino-6-methoxypyridin, 2,6-Di-hydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus 4-Hydroxyindol, 6-Hydroxyindol und 7-Hydroxyindol und deren physiologisch verträglichen Salzen.

Die erfindungsgemäß verwendbaren Indolinderivate werden bevorzugt ausgewählt aus 4-Hydroxyindolin, 6-Hydroxyindolin und 7-Hydroxyindolin und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt.

Besonders geeignet als Vorstufen naturanaloger Haarfarbstoffe geeignet sind Derivate des 5,6-Dihydroxindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und/oder 5,6-Dihydroxyindolin-2-carbonsäure. Als Vorstufen naturanaloger Haarfarbstoffe hervorragend geeignet sind weiterhin Derivate des 5,6-Dihydroxindols , insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Eine weitere Möglichkeit zur Farbveränderung bietet die Verwendung von Färbemitteln, die sogenannte Oxofarbstoffvorprodukte enthalten. Eine erste Klasse der Oxofarbstoffvorprodukte sind Verbindungen mit mindestens einer reaktiven Carbonylgruppe. Diese erste Klasse wird als Komponente (Oxo1) bezeichnet. Eine zweite Klasse der Oxofarbstoffvorprodukte bilden C,H-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind.

Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit lässt sich die Methode der Oxofärbung ohne weiteres mit dem oxidativen Färbesystem kombinieren.

In einer weiteren Ausführungsform der erfindungsgemäßen Verwendung können daher als farbverändernde Komponente daher auch Oxofarbstoffvorprodukte eingesetzt werden. Oxofarbstoffvorprodukte werden bevorzugt als Kombination aus mindestens einer Verbindung, die mindestens eine reaktive Carbonylgruppe enthält (Komponente (Oxo1)) mit mindestens einer Verbindung (Komponente Oxo2), ausgewählt aus C,H-aciden Verbindungen (Oxo2a) und/oder aus Verbindungen (Oxo2b) mit primärer oder sekundärer Aminogruppe oder Hydroxygruppe, ausgewählt aus mindestens einer Verbindung der Gruppe, die gebildet wird aus primären oder sekundären aromatischen Aminen, stickstoffhaltigen heterocyclischen Verbindungen und aromatischen Hydroxyverbindungen, eingesetzt.

Reaktive Carbonylverbindungen als Komponente (Oxo1) besitzen im Sinne der Erfindung mindestens eine Carbonylgruppe als reaktive Gruppe, welche mit der Komponente (Oxo2) unter Ausbildung einer kovalenten Bindung reagiert. Bevorzugte reaktive Carbonylverbindungen sind ausgewählt aus Verbindungen die mindestens eine Formylgruppe und/oder mindestens eine Ketogruppe, insbesondere mindestens eine Formylgruppe, tragen. Ferner sind erfindungsgemäß auch solche Verbindungen als Komponente (Oxo1) verwendbar, in denen die reaktive Carbonylgruppe derart derivatisiert bzw. maskiert ist, dass die Reaktivität des Kohlenstoffatoms der derivatisierten Carbonylgruppe gegenüber der Komponente (Oxo2) stets vorhanden ist.

Bevorzugte reaktive Carbonylverbindungen der Komponente (Oxo1) werden ausgewählt aus der Gruppe, bestehend aus Benzaldehyd, Naphthaldehyd, Zimtaldehyd und den Derivaten dieser Aldehyde, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)acetaldehyd, 1-Methylpyrrol-2-aldehyd, Pyridoxal, Antipyrin-4-aldehyd, Furfural, 5-Nitrofurfural, Chromon-3-aldehyd, 3-(5'-Nitro-2'-furyl)acrolein, 3-(2'-Furyl)acrolein und |midazo|-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)-penta-3,5-dienal, Piperonal, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, Salze, deren kationische Komponente 4-Formyl-1-methylpyridinium, 2-Formyl-1-methylpyridinium, 4-Formyl-1-ethylpyridinium, 2-Formyl-1-ethylpyridinium, 4-Formyl-1-benzylpyridinium, 2-Formyl-1-benzyl-pyridinium, 4-Formyl-1,2-dimethylpyridinium, 4-Formyl-1,3-dimethylpyridinium, 4-Formyl-1-methyl-chinolinium, 2-Formyl-1-methylchinolinium, 5-Formyl-1-methylchinolinium, 6-Formyl-1-methylchino-linium, 7-Formyl-1-methylchinolinium, 8-Formyl-1-methylchinolinium, 5-Formyl-1-ethylchinolinium, 6-Formyl-1-ethylchinolinium, 7-Formyl-1-ethylchinolinium, 8-Formyl-1-ethylchinolinium, 5-Formyl-1-benzylchinolinium, 6-Formyl-1-benzylchinolinium, 7-Formyl-1-benzylchinolinium, 8-Formyl-1-benzylchinolinium, 5-Formyl-1-allylchinolinium, 6-Formyl-1-allylchinolinium, 7-Formyl-1-allyl-chinolinium oder 8-Formyl-1-allylchinolinium ist und deren anionisches Gegenion ausgewählt aus Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat oder Tetrafluoroborat ist, Isatin, 1-Methylisatin, 1-Allylisatin, 1-Hydroxymethylisatin, 5-Chlorisatin, 5-Methoxyisatin, 5-Nitroisatin, 6-Nitroisatin, 5-Sulfoisatin, 5-Carboxyisatin, Chinisatin, 1-Methylchinisatin, sowie beliebigen Gemischen der voranstehenden Verbindungen.

Als C,H-acide Verbindungen (Oxo2a) sind solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-WasserstoffBindung bewirkt wird. Prinzipiell sind der Auswahl der C,H-aciden Verbindungen keine Grenzen gesetzt, solange nach der Kondensation mit den reaktiven Carbonylverbindungen der Komponente (Oxo1) eine für das menschliche Auge sichtbar farbige Verbindung erhalten wird. Es handelt sich erfindungsgemäß bevorzugt um solche C,H-aciden Verbindungen, welche einen aromatischen und/oder einen heterocyclischen Rest enthalten. Der heterocyclische Rest kann wiederum aliphatisch oder aromatisch sein. Besonders bevorzugt werden die C,H-aciden Verbindungen ausgewählt aus heterocyclischen Verbindungen, insbesondere kationischen, heterocyclischen Verbindungen oder Heterocyclen-haltige Acetonitrile.

Besonders bevorzugte Verbindungen (Oxo2a) werden ausgewählt aus einer oder mehrerer Verbindungen der Gruppe von Salzen mit physiologisch verträglichem Gegenion, die gebildet wird aus Salzen des 1,2-Dihydro-1,3,4,6-tetramethyl-2-oxo-pyrimidiniums, 1,2-Dihydro-1,3,4-trimethyl-2-oxo-pyrimidiniums, 1-Allyl-1,2-dihydro-3,4,6-trimethyl-2-oxo-pyrimidiniums, 1,2-Dihydro-1-(2-hydroxyethyl)-3,4,6-trimethyl-2-oxo-pyrimidiniums und 1,2-Dihydro-1,3,4,6-tetramethyl-2-thioxo-pyrimidiniums, wobei als Gegenion bevorzugt Halogenide, Benzolsulfonat, p-Toluolsulfonat, $(C_1-C_4-Alkan)$sulfonat, Trifluormethansulfonat, Perchlorat, 0,5 Sulfat, Hydrogensulfat, Tetrafluoroborat, Hexafluorophosphat oder Tetrachlorozinkat eingesetzt werden.

Weiterhin besonders bevorzugte Verbindungen (Oxo2a) sind ausgewählt aus mindestens einer Verbindung der Gruppe, bestehend aus 2-(2-Furoyl)acetonitril, 2-(5-Brom-2-furoyl)acetonitril, 3-(2,5-Dimethyl-3-furyl)-3-oxopropanitril, 2-(2-Thenoyl)acetonitril, 2-(3-Thenoyl)acetonitril, 2-(5-Fluor-2-thenoyl)acetonitril, 2-(5-Chlor-2-thenoyl)acetonitril, 2-(5-Brom-2-thenoyl)acetonitril, 2-(5-Methyl-2-thenoyl)acetonitril, 2-(2,5-Dimethylpyrrol-3-oyl)acetonitril, 2-(1,2,5-Trimethylpyrrol-3-oyl)acetonitril, 1*H*-Benzimidazol-2-ylacetonitril, 1*H*-Benzothiazol-2-ylacetonitril, 2-(Pyrid-2-yl)acetonitril, 2,6-Bis-(cyanomethyl)pyridin, 2-(Idol-3-oyl)acetonitril, 2-(2-Methyl-indol-3-oyl)acetonitril und 2-(6-Hydroxy-4,7-dimethoxy-1-benzofuran-5-oyl)acetonitril, insbesondere 1*H*-Benzimidazol-2-ylacetonitril.

Des Weiteren kann als Komponente (Oxo2b) mindestens ein Oxidationsfarbstoffvorprodukt mit mindestens einer primären oder sekundären Aminogruppe und/oder mindestens einer Hydroxygruppe verwendet werden. Bevorzugt geeignete Vertreter finden sich unter der Ausführung der Oxidationsfarbstoffvorprodukte. Es ist jedoch erfindungsgemäß bevorzugt, wenn die Verbindungen der Komponente (Oxo2) nur unter C,H-aciden Verbindungen ausgewählt werden.

Die voranstehend genannten Verbindungen der Komponente (Oxo1) sowie der Komponente (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

Die erfindungsgemäße Verwendung zur Verbesserung der Farbintensität ist weiterhin **dadurch gekennzeichnet, dass** die Färbezubereitung mindestens einen Wirkstoff eine Aminosäure und/oder deren Salze enthält.

Als Aminosäure im Sinne der Erfindung gilt eine organische Verbindung, die in ihrer Struktur mindestens eine protonierbare Aminogruppe und mindestens eine -COOH- oder eine -SO$_3$H-Gruppe enthält. Bevorzugte Aminosäuren sind Aminocarbonsäuren, insbesondere a-(alpha)-Aminocarbonsäuren und ω-Aminocarbonsäuren, wobei α-minocarbonsäuren besonders bevorzugt sind.

α-minocarbonsäuren enthalten üblicherweise mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Bevorzugt wird als Aminosäure eine hydroxylgruppen-haltigen Aminosäure und/oder deren Salz eingesetzt. Als hydroxylgruppen-haltige Aminosäure im Sinne der Erfindung gilt eine organische Verbindung, die in ihrer Struktur mindestens eine protonierbare Aminogruppe und mindestens eine -COOH- oder eine -SO$_3$H-ruppe sowie mindestens eine Hydroxyl-Gruppe enthält. Bevorzugt ist die Hydroxyl-Gruppe an einen aliphatischen Rest gebunden.

Eine weiteren Ausführungsform der erfindungsgemäßen Verwendung ist **dadurch gekennzeichnet, dass** das Mittel als hydroxylgruppen-haltige Aminosäure eine Verbindung, ausgewählt aus der Gruppe, die gebildet wird aus Serin, Threonin, 4-Hydroxyprolin und/oder deren Salzen, enthält.

Erfindungsgemäß besonders bevorzugt sind dabei Mittel, die zur Farbintensivierung Serin enthalten. Besonders bevorzugt enthält die Färbezubereitung zur Farbintensivierung Serin, D-Serin oder D/L-Serin, ganz besonders bevorzugt jedoch L-Serin, insbesondere in freier Form, aber auch als Hydrochlorid.

Die hydroxylgruppen-haltigen Aminosäuren können den erfindungsgemäßen Mitteln bevorzugt in freier Form zugegeben werden. In einer Reihe von Fällen ist es jedoch auch vorteilhaft, die Aminosäuren in Salzform einzusetzen. Bevorzugte Salze sind insbesondere Hydrochloride, Hydrobromide und Sulfate.

Die hydroxylgruppen-haltige(n) Aminosäure(n) und/oder deren Salze ist/sind in den Färbezubereitungen der erfindungsgemäßen Verwendung bevorzugt in Mengen von 0,01 bis 10 Gew.-%, insbesondere von 0,05 bis 5 Gew.-%, bezogen auf das Gesamtgewicht der Anwendungsmischung, enthalten.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Bevorzugte Oxidationsmittel sind Persulfate, Peroxodisulfate, Chlorite, Hypochlorite und insbesondere Wasserstoffperoxid oder dessen Anlagerungsprodukte an Harnstoff, Melamin sowie Natriumborat.

Eine bevorzugte Ausführungsform der erfindungsgemäßen Verwendung ist daher **dadurch gekennzeichnet, dass** das Mittel zusätzlich mindestens ein Oxidationsmittel, ausgewählt aus Wasserstoffperoxid und/oder einen seiner Anlagerungsprodukte an organische oder anorganische Verbindungen, enthält.

Bevorzugte Anlagerungsprodukte sind die Anlagerungsprodukte von Wasserstoffperoxid an Harnstoff, Melamin sowie Natriumborat. Bevorzugt wird jedoch als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 1 bis 8 Gew.-%, jeweils bezogen auf das anwendungsbereite Mittel.

Erfindungsgemäß kann das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erwiesen, wenn die Färbemittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra-(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Bei einer Anwendung von zusätzlichen Oxidationsmitteln wird die eigentliche Färbezubereitung zweckmäßigerweise unmittelbar vor der Anwendung durch Mischung einer erfindungsgemäßen Zubereitung, enthaltend mindestens eine farbverändernde Komponente und mindestens eine Aminosäure, sowie einer Zubereitung, enthaltend das zusätzliche Oxidationsmittel, insbesondere Wasserstoffperoxid, hergestellt.

Die erfindungsgemäß verwendbaren Mittel können zusätzlich Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbeund Aufhellmittel" bezeichnet. Für die starke Aufhellung sehr dunklen Haares ist jedoch der alleinige Einsatz von Wasserstoffperoxid oder dessen Anlagerungsprodukten an organische beziehungsweise anorganische Verbindungen oftmals nicht ausreichend.

Daher kann es erfindungsgemäß, sollte der Verbraucher den Wunsch nach einer sehr starken Blondierung verspüren, in einer weiteren Ausführungsform bevorzugt sein, wenn das Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Zur Steigerung der Aufhellwirkung von Wasserstoffperoxid ist ebenso möglich, anstelle von Peroxo-Salzen Bleichkraftverstärker zuzugeben. Hierzu zählen Siliciumdioxid-Verbindungen sowie insbesondere kationisierte Heterocyclen.

Zur weiteren Steigerung der Leistung der Oxidationsmittelzubereitung kann der erfindungsgemäßen Zusammensetzung daher zusätzlich mindestens eine gegebenenfalls hydratisierte $SiO_2$ Verbindung zugesetzt werden. Es kann erfindungsgemäß bevorzugt sein, die gegebenenfalls hydratisierten $SiO_2$Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der $SiO_2$-erbindungen (ohne deren Wasseranteil) in den Mitteln wieder.

Hinsichtlich der gegebenenfalls hydratisierten $SiO_2$-erbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere deren Kaliumund Natriumsalze. Die gegebenenfalls hydratisierten $SiO_2$-erbindungen können in verschiedenen Formen vorliegen. Erfindungsgemäß bevorzugt werden die $SiO_2$-erbindungen in Form von Kieselgelen (Silicagel) oder als Wasserglas eingesetzt. Erfindungsgemäß besonders bevorzugt sind Wassergläser.

Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Zur erfindungsgemäßen Verwendung besonders geeignete Färbemittel zeichnen sich dadurch aus, dass sie zusätzlich mindestens ein amphoteres und/oder kationisches Polymer enthalten.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen.

Besonders geeignet sind solche Färbemittel, die ein amphoteres oder kationisches Polymer enthalten, welches die kationische Ladung in einer Seitenkette des Polymers trägt.

Insbesondere solche Polymere, bei denen die quartäre Ammoniumgruppe über eine $C_{1-4}$Alkyengruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (I),

$$[-CH_2C(R^1)(C(_=O)O-(CH_2)_m-N^+R^2R^3R^4)-]_n \; X^- \qquad (I),$$

in der $R^1$ = -H oder -CH$_3$ ist, $R^2$, $R^3$ und $R^4$ unabhängig voneinander ausgewählt sind aus C$_{1-4}$-Alkyl-, -Alkenyl- oder -Hydroxyalkylgruppen, m = 1, 2, 3 oder 4, n eine natürliche Zahl und X$^-$ ein physiologisch verträgliches organisches oder anorganisches Anion ist, sowie Copolymere, bestehend im wesentlichen aus den in aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die gilt, dass $R^1$ für eine Methylgruppe steht, $R^2$, $R^3$ und $R^4$ für Methylgruppen stehen und m den Wert 2 hat.

Als physiologisch verträgliches Gegenionen X$^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyl-oxyethyltrimethylammoniumchlorid) mit der INCI-Bezeichnung Polyquaternium-37.

Weitere bevorzugte kationische Polymere sind beispielsweise quaternisierte Cellulose-Derivate; kationische Alkylpolyglycoside; kationisierter Honig; kationische Guar-Derivate; Polysiloxane mit quaternären Gruppen,; Copolymere des Vinylpyrrolidons mit quaternierten Derivaten des Dialkylaminoalkylacrylats und -methacrylats; Vinylpyrrolidon-Vinylimidazoliummethochlorid-Copolymere sowie quaternierter Polyvinylalkohol. Gleichfalls als kationische Polymere eingesetzt werden können die unter den Bezeichnungen Polyquaternium-24 bekannten Polymere.

Weitere im erfindungsgemäßen Verfahren einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate. Chitosane sind deacetylierte Chitine, die in unterschiedlichen Deacetylierungsgraden und unterschiedlichen Molekulargewichten im Handel erhältlich sind.

Weiterhin können als Polymere zur Steigerung der Wirkung amphotere Polymere im Färbemittel als Bestandteil eingesetzt werden. Unter dem Begriff amphotere Polymere werden sowohl solche Polymere, die im Molekül sowohl freie Aminogruppen als auch freie CO$_2$H- oder SO$_3$-Gruppen enthalten und zur Ausbildung innerer Salze befähigt sind, als auch zwitterionische Polymere, die quartäre Ammoniumgruppen und CO$_2^-$- oder SO$_3^-$-Gruppen enthalten, und solche Polymere zusammengefasst, die CO$_2$H- oder SO$_3$H-Gruppen und quartäre Ammoniumgruppen enthalten.

In einer Ausführungsform der erfindungsgemäßen Verwendung enthält das Färbemittel daher mindestens ein Copolymer, welches aufgebaut ist aus mindestens einem anionischen Monomer, ausgewählt aus Acrylsäure, Methacrylsäure und/oder deren physiologisch verträglichen Salzen und aus mindestens einem kationischen Monomer, ausgewählt aus quaternierten Alkyl Methacrylamiden und Acrylamiden vom Typ CH$_2$=C($R^1$)(C(=O)X-(CH$_2$)$_m$-N$^+$R$^2$R$^3$R$^4$ , worin X entweder für O oder für NH steht.

Bevorzugt eingesetzte amphotere Polymere sind solche Polymerisate, die sich im wesentlichen zusammensetzen aus

(a) Monomeren mit quartären Ammoniumgruppen der allgemeinen Formel (II),

$$R^5CH=CR^6CO\text{-}Z\text{-}(C_nH_{2n})\text{-}N^{(+)}R^7R^8R^9 \qquad (II),$$

in der $R^5$ und $R^6$ unabhängig voneinander stehen für Wasserstoff oder eine Methylgruppe und $R^7$, $R^8$ und $R^9$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A$^{(-)}$ das Anion einer organischen oder anorganischen Säure ist, und
(b) monomeren Carbonsäuren der allgemeinen Formel (III),

$$R^{10}\text{-}CH=CR^{11}\text{-}COOH \qquad (III),$$

in denen $R^{10}$ und $R^{11}$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, im erfindungsgemäßen Verfahren eingesetzt werden. Ganz besonders bevorzugt sind solche Polymerisate, bei denen Monomere des Typs (a) eingesetzt werden, bei denen $R^7$, $R^8$ und $R^9$ Methylgruppen sind, Z eine NH-Gruppe und A$^{(-)}$ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Besonders bevorzugt ist eine Färbezubereitung, die als amphoteres Polymer ein Copolymer aus mindestens Acrylsäure und/oder dessen physiologisch verträglichen Salzen und aus mindestens N,N,N-Trimethylammoniopropyl-Acrylamid Chlorid enthält.

Die amphoteren und/oder kationischen Polymere sind in zur erfindungsgemäßen Verwendung eingesetzten Mitteln bevorzugt von 0,01 bis 10 Gew.-%, bezogen auf das gesamte Mittel, enthalten. Mengen von 0,1 bis 5 Gew.-%, insbesondere von 0,1 bis 3 Gew.-%, sind bevorzugt.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

In einer weiteren bevorzugten Ausführungsform der vorliegenden Erfindung enthält das Färbemittel weiterhin mindestens ein amphoteres und/oder ein zwitterionisches Tensid.

Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethylimidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

In einer weiteren Ausführungsform der vorliegenden Erfindung enthält das Mittel weiterhin mindestens ein amphoteres Tensid. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Bevorzugte amphotere Tenside werden unter der INCI-Bezeichnung Disodium Cocoamphodipropionate und Disodium Cocoamphodiacetate vermarktet.

Erfindungsgemäß bevorzugt sind Färbezubereitungen, die als amphoteres oder zwitterionisches Tensid mindestens eine Verbindung, ausgewählt aus Disodium Cocoamphodiacetate, Disodium Cocoamphodipropionate und/oder Cocamidopropyl Betaine, enthalten.

Die amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Seifen, Alkylsulfate, Alkylethersulfate und Ethercarbonsäuren mit 10 bis 18 C-Atomen in der Alkylgruppe und bis zu 12 Glykolethergruppen im Molekül.

Weiterhin hat es sich als vorteilhaft erwiesen, wenn die erfindungsgemäßen Aufhellmittel nichtionogene grenzflächenaktive Stoffe enthalten. Solche Verbindungen sind beispielsweise Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C2-C6-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; C12-C30-Fettsäuremono- und -diester von Anlagerungsprodukten von 1 bis 30 Mol Ethylenoxid an Glycerin; Polyglycerinester und alkoxylierte Polyglycerinester; Anlagerungsprodukte von 5 bis 60 Mol Ethylenoxid an Rizinusöl und gehärtetes Rizinusöl; Polyolfettsäureester; ethoxylierte Mono-, Diund Triglyceride; alkoxylierte Fettsäurealkylester; Aminoxide; Hydroxymischether; Sorbitanfettsäureester und Anlagerungeprodukte von Ethylenoxid an Sorbitanfettsäureester wie Polysorbate; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide sowie Alkylpolyglykoside.

Die anionischen oder nichtionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Ferner können die erfindungsgemäßen Mittel weitere Wirk-, Hilfs- und Zusatzstoffe enthalten, wie beispielsweise nichtionische Polymere; zwitterionische und amphotere Polymere; anionische Polymere; Verdickungsmittel; haarkonditionierende Verbindungen; Proteinhydrolysate pflanzlicher oder tierischer Herkunft; Parfümöle, Dimethylisosorbid und Cyclodextrine; faserstrukturverbessernde Wirkstoffe; Entschäumer wie Silikone; Farbstoffe zum Anfärben des Mittels; Antischuppenwirkstoffe; Lichtschutzmittel; Wirkstoffe (Pantolacton, Allantoin, Pyrrolidinoncarbonsäuren und deren Salze sowie Bisabolol); Vitamine, Provitamine und Vitaminvorstufen; Pflanzenextrakte; pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl); Cholesterin; Konsistenzgeber; Fette und Wachse (Fettalkohole, Bienenwachs, Montanwachs und Paraffine); Quell- und Penetrationsstoffe; Trübungsmittel; Perlglanzmittel; Treibmittel Antioxidantien.

Die Auswahl dieser weiteren Stoffe wird der Fachmann gemäß den gewünschten Eigenschaften der Zubereitungen treffen. Die erfindungsgemäß eingesetzten Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Schließlich ist ein weiteres bevorzugtes Alkalisierungsmittel Ammoniak. Bevorzugt werden Acidificierungsmittel und Alkalisierungsmittel jeweils in einer Menge von 0,05 bis 15 Gew.-%, insbesondere von 0,5 bis 10 Gew.-%, bezogen auf das Gesamtgewicht des anwendungsbereiten Mittels, eingesetzt.

Ein zweiter Gegenstand der vorliegenden Erfindung ist eine Mehrkomponentenverpackungseinheit (Kit-of-Parts),
umfassend mindestens einen ersten Container (C1) mit einer Färbezubereitung (A), enthaltend in einem kosmetischen Träger mindestens ein Oxidationsfarbstoffvorprodukt, mindestens ein amphoteres oder kationisches Polymer und/oder mindestens ein amphoteres oder zwitterionisches Tensid und mindestens eine hydroxylgruppen-haltige Aminosäure und/oder deren Salze,
und mindestens einen zweiten Container (C2) mit einer Entwicklerzubereitung (B), enthaltend in einem kosmetischen Träger mindestens ein Oxidationsmittel,
mit der Massgabe, dass das amphotere oder kationische Polymer die kationische Ladung in einer Seitenkette des Polymers trägt.

Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Färbezubereitung können in einem einzelnen Container enthalten sein, es ist aber auch möglich und gegebenenfalls bevorzugt, sie auf verschiedene Behälter aufzuteilen und den Verbraucher anzuleiten, sie vor der Anwendung miteinander zu mischen.

Eine bevorzugte Ausführungsform des Erfindungsgegenstands ist eine Mehrkomponentenverpackungseinheit, welche dadurch gekennzeichnet ist, dass die Färbezubereitung (A) als hydroxylgruppen-haltige Aminosäure L-Serin enthält.

Eine weitere bevorzugte Ausführungsform dieses Erfindungsgegenstands ist eine Mehrkomponentenverpackungseinheit, die dadurch gekennzeichnet ist, dass in der Färbezubereitung (A) als amphoteres oder kationisches Polymer mindestens ein Copolymer enthalten ist, welches aufgebaut ist aus mindestens einem anionischen Monomer, ausgewählt aus Acrylsäure, Methacrylsäure und/oder deren physiologisch verträglichen Salzen und aus mindestens einem kationischen Monomer, ausgewählt aus quaternierten Alkyl Methacrylamiden und Acrylamiden.

Besonders bevorzugt ist dabei eine Mehrkomponentenverpackungseinheit, die dadurch gekennzeichnet ist, dass die Färbezubereitung (A) als amphoteres Polymer ein Copolymer aus mindestens Acrylsäure und/oder dessen physiologisch verträglichen Salzen und aus mindestens N,N,N-Trimethylammoniopropyl-Acrylamid Chlorid enthalten ist.

Eine weitere bevorzugte Ausführungsform dieses Erfindungsgegenstands ist eine Mehrkomponentenverpackungseinheit, die dadurch gekennzeichnet ist, dass die Färbezubereitung (A) als ein amphoteres oder zwitterionisches Tensid mindestens eine Verbindung, ausgewählt aus Disodium Cocoamphodiacetate, Disodium Cocoamphodipropionate und/oder Cocamidopropyl Betaine, enthält.

In einer besonders bevorzugten Ausführungsform ist die Verpackungseinheit des zweiten Erfindungsgegenstands **dadurch gekennzeichnet, dass** sie mindestens eine zusätzliche Komponente, ausgewählt aus der Gruppe, die gebildet wird aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand.

Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Im Rahmen der Verwendung wird das anwendungsbereite Färbemittel durch Vermischen der Färbezubereitung (A) mit der Entwicklerzubereitung (B) der Mehrkomponentenverpackungseinheit hergestellt, auf die keratinischen Fasern aufgetragen und für eine bestimmte Einwirkzeit im Haar belassen. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbezubereitung mit einer Applikationshilfe auf die keratinischen Fasern aufzutragen.

Die Auftragungs- und die Einwirktemperatur der Färbezubereitung beträgt Raumtemperatur bis 45°C. Gegebenenfalls kann die Wirkung der Färbezubereitung durch externe Wärmezufuhr, wie beispielsweise mittels einer Wärmehaube, verstärkt werden. Die bevorzugte Einwirkdauer der Färbezubereitung auf die keratinische Faser beträgt 2 bis 45 min, bevorzugt 5 bis 35 min. Nach Beendigung der Einwirkdauer wird das verbliebene Färbemittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet.

Die bevorzugten Ausführungsformen des ersten Erfindungsgegenstands gelten mutatis mutandis für die erfindungsgemäße Verpackungseinheit des zweiten Erfindungsgegenstandes. Die nachfolgenden Beispiele sollen bevorzugte Ausführungsformen der Erfindung erläutern, ohne sie jedoch einzuschränken.

**Beispile**

1. Zubereitungen

1.1 Herstellung der Färbezubereitung

Aus den aufgelisteten Bestandteilen (jeweils in Gew.-%) wurden Färbecremes wie folgt hergestellt

| | V1 | E1 | V2 | E2 |
|---|---|---|---|---|
| Farbstoffvorprodukte A* | 1,32 | 1,32 | - | - |
| Farbstoffvorprodukte B** | - | - | 2,28 | 2,28 |
| Lanette D | 6,60 | 6,60 | 8,50 | 8,50 |
| Lorol, C12-C18 techn. | 2,40 | 2,40 | 2,00 | 2,00 |
| Eumulgin B2 | 0,60 | 0,60 | 0,75 | 0,75 |
| Eumulgin B1 | 0,60 | 0,60 | - | - |
| Dehyton K | - | - | 12,50 | 12,50 |
| Lamesoft PO 65 | 2,00 | 2,00 | - | - |
| Akypo Soft 45HP | 10,00 | 10,00 | - | - |
| Texapon K14 S Special | 2,80 | 2,80 | 15,00 | 15,00 |
| Produkt W 37194 | 3,75 | 3,75 | - | - |
| Ammoniumsulfat, techn. rein | 0,47 | 0,47 | - | - |

(fortgesetzt)

| | V1 | E1 | V2 | E2 |
|---|---|---|---|---|
| Monoammoniumphosphat | - | - | 0,80 | 0,80 |
| Natriumsulfit, wasserfrei, 96% | 0,40 | 0,40 | 0,40 | 0,40 |
| Ascorbinsäure | 0,10 | 0,10 | 0,40 | 0,40 |
| HEDP, 60% | 0,20 | 0,20 | 0,20 | 0,20 |
| Natriumsilikat 40 / 42 | 0,50 | 0,50 | 0,50 | 0,50 |
| L-Serin | - | 1,00 | - | 1,00 |
| Phenoxyethanol | - | - | 0,60 | 0,60 |
| L-Arginin | - | - | 1,00 | 1,00 |
| Ammoniak, 25% | 6,50 | 6,50 | 1,50 | 1,50 |
| Parfum, Konservierungsmittel | qs | qs | qs | qs |
| Wasser | ad 100 | ad 100 | ad 100 | ad 100 |

*Oxidationsfarbstoffvorproduktmischung, enthaltend jeweils als Gewichtsanteil zu 9,5 Gew.-% p-Toluylendiaminsulfat, zu 4,3 Gew.-% 4,5-Diamino-1-(2-hydroxyethyl)pyrazolsulfat, zu 4,5 Gew.-% 2-(4-Methyl-2-nitrophenyl)aminoethanol, zu 5,3 Gew.-% 4-Chlorresorcin, zu 30,3 Gew.-% 5-Amino-2-methylphenol, zu 6,5 Gew.-% 2,7-Dihydroxynaphthalin und zu 39,6 Gew.-% Bis-(5-amino-2-hydroxyphenyl)methan Dihydrochlorid.
**Oxidationsfarbstoffvorproduktmischung, enthaltend jeweils als Gewichtsanteil zu 70,1 Gew.-% p-Toluylendiaminsulfat, zu 8,6 Gew.-% 4-Chlorresorcin, zu 1,5 Gew.-% 3-Aminophenol und zu 19,8 Gew.-% 2-Methylresorcin.

| | |
|---|---|
| Lanette® D | INCI-Bezeichnung: Cetearyl alcohol (Cognis) |
| Lorol® tech. | INCI-Bezeichnung: Coconut alcohol (Cognis) |
| Eumulgin® B2 | INCI-Bezeichnung: Ceteareth-20 (Cognis) |
| Eumulgin® B1 | INCI-Bezeichnung: Ceteareth-12 (Cognis) |
| Lamesoft® PO 65 | INCI-Bezeichnung: Coco-Glucoside, Glyceryl Oleate, Aqua (ca. 66 %,) (Cognis) |
| Akypo® Soft 45HP | INCI-Bezeichnung: Sodium Laureth-6 Carboxylate, Aqua (ca. 21 %,) (KAO) |
| Texapon® K14 S Special | INCI-Bezeichnung: Sodium Myreth Sulfate, Aqua (ca. 27 %) (Cognis) |
| Produkt W 37194 | INCI-Bezeichnung: Acrylamidopropyltrimonium Chloride/Acrylates Copolymer, Aqua (ca. 20 %) (Stockhausen) |
| Dehyton K | INCI-Bezeichnung: Aqua, Cocamidopropyl Betaine (ca. 30 %) (Cognis) |
| Natriumsilikat 40/42 | Natronwasserglas |

Lanette D, Lorol und Eumulgin B2 sowie ggf. Eumulgin B1 und Lamesoft PO 65 wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Die Rezeptur E1 ist einerfindungsgemäßes Beispiel. Bei der Rezeptur V1 handelt es sich um eine nicht erfindungsgemäße Vergleichsrezeptur.

1.2. Vermischen mit der Entwicklerdispersion EW

Jede Färbecreme wurde unmittelbar vor der Applikation auf die Strähnen im Gewichtsverhältnis 1:1 1 mit einer wie folgt zusammengesetzten Entwicklerdispersion ausgemischt.

| Rohstoff | Gew.-% |
|---|---|
| Ammoniak, 25 Gew.-% | 0,65 |
| Dipicolinsäure | 0,10 |
| Dinatriumpyrophosphat | 0,03 |
| HEDP, 60% | 1,50 |
| Texapon NSO | 2,00 |
| Dow Corning DB 110 A | 0,07 |
| Aculyn 33A | 15,00 |
| Wasserstoffperoxid 50 % | 12,00 |
| Wasser | ad 100 |

Texapon® NSO          INCI-Bezeichnung: Sodium Laureth Sulfate (ca. 27,5%) (Cognis)
Aculyn® 33 A          INCI-Bezeichnung: Acrylates Copolymer (ca. 28%) (Rohm & Haas)
Dow Corning® DB 110 A   INCI-Bezeichnung: Dimethicon (Dow Corning)

2. Applikation

Für die Färbung wurde auf Strähnen aus Büffelbauchhaar jeweils die 4-fache Menge der fertigen Anwendungsmischung appliziert. Nachdem die Strähnen für 30 Minuten bei 32°C gefärbt wurden, wurden sie mit einem warmen Wasser (32°C) ausgewaschen.
Die farbmetrischen Messungen erfolgten auf der Strähne an jeweils 4 Messpunkten. Als Messgerät diente der Spectralflash SF 450 der Firma Datacolor.
Die Ergebnisse der Messungen wurden mithilfe des CIELAB Farbenraums quantifiziert.
Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ΔE-Wertes charakterisiert, der entsprechend Gleichung (1) berechnet wird:

$$\Delta E = \sqrt{(\Delta L)^2 + (\Delta a)^2 + (\Delta b)^2} \qquad (I)$$

Folgende Färbeergebnisse wurden erhalten:

| Rezeptur | L* | a* | b* | ΔE |
|---|---|---|---|---|
| E1 | 22,7 | 16,7 | 7,4 | 2,0 |
| V1 | 23,1 | 16,4 | 5,5 | |
| E2 | 21,5 | 5,4 | 7,7 | 1,8 |
| V2 | 19,9 | 5,1 | 7,0 | |

Die Verwendung der erfindungsgemäßen Färbemittel führte zu einer deutlichen Verbesserung der Farbintensität ΔE -Wert) der Färbung.

**Patentansprüche**

1. Kosmetische Verwendung eines Mittels zur Intensivierung der Farbveränderung keratinischer Fasern, **dadurch gekennzeichnet, dass** das Mittel in einem kosmetischen Träger mindestens eine farbverändernde Komponente und mindestens eine Aminosäure enthält.

2. Verwendung nach Anspruch 1, **dadurch gekennzeichnet, dass** das Mittel als farbverändernde Komponente mindestens ein Oxidationsfarbstoffvorprodukt enthält.

3. Verwendung nach einem der Ansprüche 1 oder 2, **dadurch gekennzeichnet, dass** das Mittel zur Intensivierung der Farbveränderung keratinischer Fasern mindestens eine hydroxylgruppen-haltige Aminosäure und/oder deren Salze enthält.

4. Verwendung nach Anspruch 3, **dadurch gekennzeichnet, dass** das Mittel als hydroxylgruppen-haltige Aminosäure eine Verbindung, ausgewählt aus der Gruppe, die gebildet wird aus Serin, Threonin, 4-Hydroxyprolin und/oder deren Salzen, enthält.

5. Verwendung nach einem der Ansprüche 3 oder 4, **dadurch gekennzeichnet, dass** das Mittel als hydroxylgruppen-haltige Aminosäure L-Serin enthält.

6. Verwendung nach einem der Ansprüche 3 bis 5, **dadurch gekennzeichnet, dass** das Mittel eine oder mehrere hydroxylgruppen-haltige Aminosäuren und/oder deren Salze in einem Gesamtgewicht von 0,01 bis 10 Gew.-%, insbesondere von 0,01 Gew.-% bis 5 Gew.-%, bezogen auf das Gesamtgewicht des anwendungsbereiten Mittels, enthält.

7. Verwendung nach einem der Ansprüche 1 bis 6, **dadurch gekennzeichnet, dass** das Mittel zusätzlich mindestens ein Oxidationsmittel, ausgewählt aus Wasserstoffperoxid und/oder einen seiner Anlagerungsprodukte an organische oder anorganische Verbindungen, enthält.

8. Mehrkomponentenverpackungseinheit,
umfassend mindestens einem ersten Container (C1) mit einer Färbezubereitung (A), enthaltend in einem kosmetischen Träger

   a) mindestens ein Oxidationsfarbstoffvorprodukt
   b) mindestens ein amphoteres oder kationisches Polymer und/oder mindestens ein amphoteres oder zwitterionisches Tensid
   c) mindestens eine hydroxylgruppen-haltige Aminosäure und/oder deren Salze,

   und mindestens einen zweiten Container (C2) mit einer Entwicklerzubereitung (B),
   enthaltend in einem kosmetischen Träger mindestens ein Oxidationsmittel,
   mit der Massgabe, dass das amphotere oder kationische Polymer die kationische Ladung in einer Seitenkette des Polymers trägt.

9. Mehrkomponentenverpackungseinheit nach Anspruch 8, **dadurch gekennzeichnet, dass** die Färbezubereitung (A) als hydroxylgruppen-haltige Aminosäure L-Serin enthält.

10. Mehrkomponentenverpackungseinheit nach einem der Ansprüche 8 oder 9, **dadurch gekennzeichnet, dass** in der Färbezubereitung (A) als amphoteres oder kationisches Polymer mindestens ein Copolymer enthalten ist, welches aufgebaut ist aus mindestens einem anionischen Monomer, ausgewählt aus Acrylsäure, Methacrylsäure und/oder deren physiologisch verträglichen Salzen und aus mindestens einem kationischen Monomer, ausgewählt aus quaternierten Alkyl Methacrylamiden und Acrylamiden.

11. Mehrkomponentenverpackungseinheit nach einem der Ansprüche 8 bis 10, **dadurch gekennzeichnet, dass** die Färbezubereitung (A) als amphoteres Polymer ein Copolymer aus mindestens Acrylsäure und/oder dessen physiologisch verträglichen Salzen und aus mindestens N,N,N-Trimethylammoniopropyl-Acrylamid Chlorid enthalten ist.

12. Mehrkomponentenverpackungseinheit nach einem der Ansprüche 8 bis 11, **dadurch gekennzeichnet, dass** die Färbezubereitung (A) als ein amphoteres oder zwitterionisches Tensid mindestens eine Verbindung, ausgewählt aus Disodium Cocoamphodiacetate, Disodium Cocoamphodipropionate und/oder Cocamidopropyl Betaine, enthält.